# EUROPEAN PATENT APPLICATION

(11) **EP 0 916 621 A2**
(43) Date of publication of application: **19.05.1999**
(21) Application number: 98120550.3
(22) Date of filing: 30.10.1998
(51) Int. Cl.: C02F 1/48, A23L 2/00, A61K 7/00, A61K 33/00, G03C 5/30, C04B 22/00

(54) **Water having a superior ability to disperse oil and fats**

(30) Priority: 30.10.1997 JP 316158/97
(71) Applicant: Hattori, Toshimitsu, Fukuoka, 819-0025 (JP)
(72) Inventor: Hattori, Toshimitsu, Fukuoka, 819-0025 (JP); Matsushita, Kazuhiro, Tokyo 196-0025 (JP)
(74) Representative: Bühling, Gerhard, Dipl.-Chem.

(57) **Abstract**

Fine-clustered water made from pure water is obtained by fine clustering treatment of pure water and is able to disperse at least about 1.5 times as much glyceryl trioleate as purified water is.

The water of the present invention can be used in pharmaceutical compositions, food compositions, cosmetic compositions, eye drop compositions, eye wash compositions, contact lens cleaner, agricultural chemical compositions, photo developing solution, and concrete compositions.

Especially, when the fine-clustered water of the present invention is used in pharmaceutical compositions, not only are the drugs dissolved easily in the water, but they are also rapidly absorbed via and delivered to tissue (oral mucosa, skin, mesentery) as compared with the purified water.

## Description

### BACKGROUND OF THE INVENTION

### 1.FIELD OF THE INVENTION:

The present invention relates to water which has a superior ability to disperse oil and fats. More specifically, the present invention relates to water which can disperse at least about 1.5 times as much glyceryl trioleate (triolein) as pure water can; the use of the water; and the method for producing the water.

### 2.DESCRIPTION OF THE RELATED ART:

Water is an important substance which is applicable to various field of industry. Especially, in the fields of pharmaceuticals, foods, cosmetics or detergents, water is mainly used as a solvent. In order to dissolve or disperse a substance (which is poorly soluble or insoluble in a solvent) in water, or in order to prepare an emulsion, a water-soluble solvent or surfactant must be added to water.

In the fields of pharmaceuticals, foods and cosmetics, although the use of solvents or surfactants is undesirable in view of safety, there is no specific proposal to use smaller amounts of solvents or surfactants. Therefore, a technique of dissolving or dispersing a water-insoluble substance in water without using a solvent or detergent has been eagerly desired.

In view of the above, the present inventors have conducted extensive studies on water and found out that the fine-clustered water from pure water can improve the solubility or dispersing ability of oil and fats, and thus accomplished the present invention.

### SUMMARY OF THE INVENTION

According to the present invention, water which is able to disperse at least about 1.5 times as much glyceryl trioleate as purified water can, is provided, and thus the present invention can solve the above-mentioned problems.

The present invention relates to fine clustered water from pure water which is obtained by a fine clustering treatment of pure water and is able to disperse at least about 1.5 times as much glyceryl trioleate as purified water is.

In a preferred embodiment, the fine-clustered water from pure water of the present invention is able to disperse at least about 2 times as much glyceryl trioleate as purified water is.

In a preferred embodiment, the fine-clustered water from pure water of the present invention is obtained by a fine clustering treatment of pure water.

In a further preferred embodiment, the fine-clustered water from pure water is obtained by treating pure water with magnetic field.

Another invention is a method for producing the fine-clustered water from pure water which is able to disperse at least about 1.5 times as much glyceryl trioleate as purified water can, wherein the method comprises the step of a fine clustering treatment of pure water.

In a preferred embodiment, the fine clustering treatment is a treatment with magnetic field.

Yet another invention is a composition which comprises the fine-clustered water from pure water which is obtained by the fine clustering treatment of pure water and is able to disperse at least about 1.5 times as much glyceryl trioleate as purified water is.

In a preferred embodiment, the fine-clustered water from pure water comprised in the composition is able to disperse at least about 2 times as much glyceryl trioleate as purified water is.

In a preferred embodiment, the fine-clustered water from pure water is obtained by fine clustering treatment of pure water.

In a preferred embodiment, the fine-clustered water from pure water is obtained by treating the pure water with magnetic field.

In a further preferred embodiment, the composition can be a pharmaceutical composition, cosmetic composition, food composition, eye drop composition, eye wash composition, contact lens cleaner, agricultural chemical composition, photo developing solution, or concrete composition.

In a preferred embodiment, the absorption speed of a drug in the pharmaceutical composition via transdermal, mucosa or mesentery is faster than that of the case where the drug is in a pharmaceutical composition based on purified water.

In a further preferred embodiment, the present pharmaceutical composition is able to carry a drug faster than that of the case where the drug is in purified water.

In a preferred embodiment, the composition can be a food composition for beverage use.

In a preferred embodiment, the composition can be a cosmetic composition selected from the group consisting of cosmetic creams, cosmetic lotions, foundation, shampoo, treatments, and hair liquid.

### BRIEF DESCRIPTION OF THE DRAWINGS

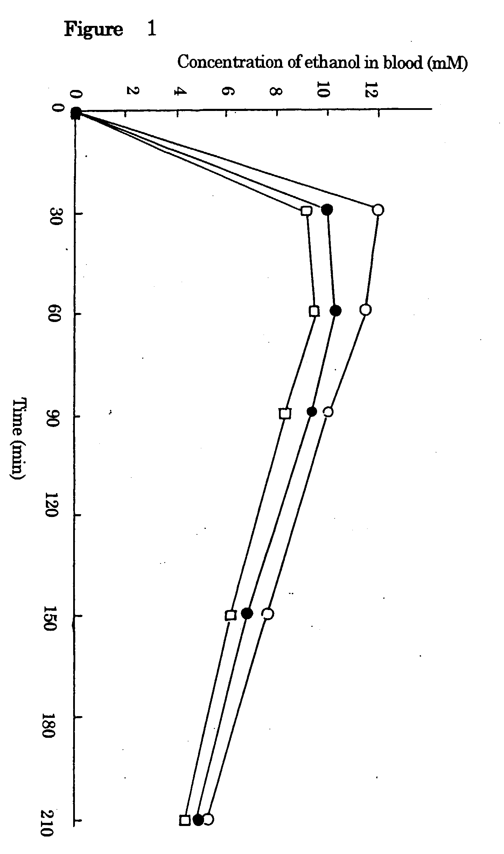

Figure 1 shows the time course of ethanol concentration in the blood of a rat when ethanol is dissolved in the fine-clustered water from pure water, the fine-clustered water from purified water, and purified water, respectively.

### DESCRIPTION OF THE PREFERRED EMBODIMENTS

The fine-clustered water from pure water of the present invention is obtained by conducting a fine clustering treatment of pure water.

The word "pure water" in the present specification means water obtained by desalting water, which is substantially pure and has a specific resistance of about 50Ω · cm or more. Ultra pure water can also be included. The "purified water" in the present invention means water obtained by distillation or ion exchange treatment.

The word "cluster" means an aggregate of water molecules. The aggregate is formed by electric forces between water molecules (i.e., the hydrogen bond between oxygen and hydrogen). In the present specification, a fine clustering treatment means reducing the size of the cluster.

The water which is used in the fine clustering treatment includes pure water, purified water (defined by Japanese Pharmacopoeia), service water, ion-exchanged water, and so on. Water other than pure water can be made into pure water by using any suitable method, such as ion-exchange treatment, distillation, ultrafiltration, reverse osmosis and any combination thereof. In the present invention, by conducting a fine clustering treatment of pure water, water having superior characteristics can be obtained. The water obtained by fine clustering treatment of pure water has not only a high ability to disperse oil and fats, but also improves the absorbability of a dissolved or dispersed substance. As a result, the water of the present invention has excellent drug delivery ability, which is not found in known water. Since purified water, service water, ion-exchanged water and so on may contain minerals (e.g., metal ions), the size of the cluster of water may not become sufficiently small.

The fine-clustered water from pure water of the present invention can be obtained by treating pure water with magnetic field, very weak electric current, an electric field, ceramic plates, far infrared, high-frequency waves (electromagnetic waves), mechanical treatment, ultrasound, electromotive force, laser beams, or natural ore. The treatment can be used alone or in combination.

Treatment with magnetic field can be performed by passing pure water with a certain speed (e.g., from about 10 to 20m/min) through a magnet apparatus. The magnet apparatus has magnets defining a gap having a magnetic field strength of about 0.07T or more (T: tesla; 1 tesla is 10,000gauss), and water is passed through the gap. Preferably, the treatment is performed using magnetic field 0.3T or more; most preferably 1.2T or more.

The treatment using very weak electric current can be conducted by applying very weak electric current with several ten milliamperes to pure water running through a gap between an uninsulated internal electrode and an external electrode.

The treatment using an electric field can be conducted by applying a DC or AC voltage of 1,000V or more to pure water in a storage tank. In order to increase the electric conductivity, for example, charcoal can be immersed in the storage tank.

The treatment using ceramic plates can be done by immersing ceramic plates in pure water in a storage tank. The ceramic plates can be made from materials which can irradiate far infrared having a wavelength from about 6 to 14 µm.

The treatment with far infrared includes a method of passing pure water through a ceramic filter which can irradiate far infrared, or a method of irradiating pure water with a far infrared heater. The principles of the method using the ceramic filter are substantially the same as that of the treatment with ceramic plate.

The treatment with high frequency waves (electro-magnetic waves) is a method of applying waves having a frequency higher than that of ultrasonic waves to pure water. This treatment includes microwave treatment, such as by electronic oven.

The mechanical treatment includes a method of rotating pure water at high speed.

The treatment with ultrasonic waves is to irradiate pure water with ultrasonic waves having a frequency from 20 to 40 KHz. As for the ultrasonic waves, both high power and low power can be used. The high power ultrasonic waves can be used for cleaning jewels or glassware. The low power ultrasonic waves can be used for maturing Japanese sake and spirits.

The electromotive force treatment is a method for making the cluster of the water small by using the difference of an ionization voltage. The difference of the ionization voltage is produced by passing pure water through the path defined by different electric conductive materials.

The laser beam treatment can be performed by using various media that emit laser beams. For example, water can be treated to irradiation with a gas laser (from 109.8nm of H₂ to 1990.75nm of CDCl₃), liquid laser, or solid laser (YAG laser, glass laser, ruby laser and so on).

The treatment using natural ore is a method of passing pure water through layers of natural ore with an appropriate pressure so as to make the cluster of the water small.

Hereinafter, a method for preparing the fine-clustered water of the present invention by using magnetic field will be described. However, the method of the present invention should not be limited to that of using magnetic field. The treatment with magnetic field is preferably conducted by using a water tank or water channel formed from non-magnetic material, which cross section having a slit-like structure. A pair of magnets (magnetic force generator) are disposed on the longitudinal sides of the cross section of the water tank or the water channel so as to allow the different magnetic poles of the magnets to face each other, and the water is circulated through the water tank or the water channel.

According to the method of treating pure water with magnetic field, the pure water can be treated by irradiating the water stream with magnetic field, generated from the magnetic force generator, which are substantially perpendicular to the water stream. As the magnetic generator, both permanent magnets and electromagnets can be used. The permanent magnets are preferable in view of changing the magnets, and the electromagnet is preferable in view of being capable of easily generating high-power magnetic field.

Examples of apparatus for producing the fine-clustered water are as follows:
(1) an apparatus comprising:
   a water tank for storing water to be treated;
   a pump which sends the water from the tank;
   a water circulating channel for returning the water to the tank;
   a magnetic force generator disposed in the middle of the water-circulating channel for generating magnetic field substantially perpendicular to the water stream: or
(2) The apparatus of above (1), wherein a plurality of magnetic force generators are disposed in the water circulating channel in series or parallel.

Preferably, the magnetic force generator disposed in the apparatus comprises:
a water channel main body constituting a part of the water-circulating channel and being formed from a non-magnetic material, which cross section has a slit;
magnets being disposed on the longitudinal sides of the cross section so as to allow different magnetic poles of the magnets to face each other; and
pressing plates that hold the water channel main body and the magnets as one body.

The width of the slit (water channel) is not limited; however, preferably, from about 0.1 mm to about 20 mm, more preferably, from about 0.5 mm to about 10 mm, or further preferably, about 2 mm.

The temperature of the pure water to be treated and the pressure of treatment are not limited. However, preferably, the pure water is irradiated with magnetic field having an intensity from about 0.07T to about 15T; preferably, from about 0.3T to about 3T; and more preferably, from about 0.8T to about 2T. For example 1.2T at a temperature from about 10 to 30°C, for example, about at 25°C, under atmospheric pressure, for about 5 to 120 hours and preferably about 10 to 50 hours, for example, 24 hours.

Further, the magnets can be disposed in the water tank so that the different magnetic poles (N-S) of the magnets face each other and the gap between the magnets is filled with the pure water so that the pure water can be treated with magnetic field.

A general example of apparatus and method for preparing magenet-treated water is disclosed in Japanese Laid Open Patent Publication No. 8-197064.

The dispersing ability of the thus-obtained water can be measured by the following method. To each 10ml of the fine-clustered water and purified water, 43.5mg of glyceryl trioleate (Wako pure chemicals: purity 99% or more) is added, followed by shaking with 128rpm, for 5 minutes. Immediately after the shaking, 4 ml of the sample is recovered. A portion of the sample, 0.54ml, is put in a NMR sample tube of 5mm diameter. As an internal standard (standard material), 0.06ml of 10mM TSP-d₄ solution in heavy water is added. After adjusting the final concentration of TSP-d₄ to 1mM, ¹H-NMR measurement is performed. The concentration of glyceryl trioleate dispersed in the water can be calculated from the ratio of the signal intensity of the methylene group of the glyceryl trioleate and the methyl proton of TSP-d₄.

As purified water, purified water (made by Wako pure chemicals, Japan) which conforms with the Japanese Pharmacopoeia is used.

The fine-clustered water from pure water of the present invention has not only a high dispersing ability of oil and fats, but also improves the absorbability of a dissolved or dispersed substance; that is, the fine-clustered water from pure water of the present invention has a good drug delivery ability, which is not found in known water. Since the pure water which is a starting material of the present water contains few minerals (metal ions) which are usually contained in purified water, the size of the cluster of the fine-clustered water from pure water tend to be small. Furthermore, the resultant small sized clusters do not tend to aggregate to form largeclusters. Therefore, the fine-clustered water from pure water of the present invention is believed to have a higher dispersing ability compared with the water obtained by purified water using a fine clustering treatment. According to these features, the water of the present invention can be used preferably in pharmaceuticals, cosmetics, foods and so on.

Various uses of the water of the present invention will be described. The water of the present invention was found to have not only a mere humective effect which prevents water from evaporating from the skin, but also to have excellent absorbability and permeation to skin tissues. Therefore, when the water of the present invention is used as a solvent for percutaneous absorbents or mucosal absorbents, these pharmaceuticals are dissolved easily in the water and are rapidly absorbed by tissue as compared to usual water (purified water). As a result, the effect that pharmaceuticals can be delivered rapidly is attained. For example, rapid absorption and delivery of drugs (pharmaceuticals) from (oral) mucosa, skin, or mesentery can be obtained. Further, rapid delivery of water and pharmaceuticals into blood can be obtained.

The fine-clustered water from pure water of the present invention can be used for pharmaceutical preparations and can attain the above-mentioned effects. The fine-clustered water from pure water of the present invention can be used as a water component of, for example, an emulsifying agent, a dispersing agent and a solubilizing agent for internal and external medicine; a water soluble base or emulsifying base for ointments or suppositories; a liposome; an absorption-promoting agent for hard-absorbing medicine; an anesthetic medicine; a composition for artificial dialysis; a diagnostic agent; a composition for artificial blood; in eye drops and so on. Further, for example, the fine-clustered water from pure water of the present invention can be used to extract effective components of Chinese herbal remedies and so on. The fine-clustered water from pure water of the present invention can also be used for activation of the various reactions in a biological body.

In cosmetics, the fine-clustered water from pure water of the present invention can be used in cleaning cosmetics (cleansing creams, degreasers, soap, antibacterial soap and so on); fundamental cosmetics (cosmetic lotions, creams, milk lotions, face packs and so on); makeup cosmetics (foundation, lipsticks, cheek rouge, face powder and so on); cosmetics for hair (hair tonic, hair liquid and so on); bath soap or body care cosmetics (soap, body shampoo, shampoo, rinse, hair treatments and so on); and oral care cosmetics (tooth powder/paste, mouth wash and gargle).

For food compositions, the fine-clustered water from pure water of the present invention can be used in emulsified food, oily food, soup (for example, Chinese noodles), emulsifiers, dispersing agents, stabilizing agents for emulsified food, antifoaming agents, viscosity decreasing agents, anti-separation agents for whey, spreaders, film forming /or glazing agents on the epidermis of fruit and/or vegetables, stabilizing agents, anti-aging agents, anti-sticking agents, air holding agents, anti-brooming agents, dough adjusting and improving agents, decreasers for absorption of oil, dispersing agents for essence or spice, tissue improving agents, sun exposure improvers, solubilizers, flexibility improving agents, capacity increasing agents, whipping agents, soft drinks, beverages or juices (for example, canned coffee, concentrated vegetable juice and so on), potable water and so on.

The fine-clustered water from pure water of the present invention can be used, for example, as a reagent for research purpose or for eye drop compositions, eye wash compositions, and contact lens cleaner; and can be used in the textile industry, agricultural, forestry and fishery industries, paper and pulp industries, cleaning industry, metal/machinery/noble metal/jewel industries, mining industry, leather industry, rubber/plastic industry, coloring material industry (such as pigments, paint, and ink), civil engineering/construction industry, ceramic industry, coal industry, petroleum/fuel industry, photographic industry, fermentation industry and so on.

Regarding the reagents for research purpose, the fine-clustered water from pure water of the present invention can be used in reagents for genetic engineering and protein engineering, reagents for extracting biological materials, reagents for organic synthesis, reagents for various analysis and so on.

Regarding the eye drop composition, the fine-clustered water from pure water of the present invention can be used in agents for the treatment of eye disease and so on. For example, eye wash can be used.

Regarding the contact lens cleaner, the fine-clustered water from pure water of the present invention can be used in both soft and hard contact lens cleaners.

In the textile industry, the fine-clustered water from pure water of the present invention can be used in, for example, spinning oil agents, knitting oil, sizing oil, de-gumming cleaners, marcerizing agents, alkaline treatment agents for polyester fiber, bleaching agents, carbonizing agents, milling agents, dyeing assistant auxiliaries, dye dispersing agents, softening agents for bathing, soaping agents after dyeing, decolorizing agents, finishing agents, softening agents, softening agents for resin processing, softening agents for water repellent, water and oil repellents, stain-proofing agents, anti-static agents, browning inhibitors and so on.

In the agricultural, forestry and fishery industries, the fine-clustered water from pure water of the present invention can be used in agricultural chemicals, emulsified agricultural chemicals, consolidation inhibitors, powdered chemicals, spreaders, additives for fertilizer, antifoaming agents, plant growth regulating preparations, canning, water for cultivation, water purifiers (for the activation of aerobic bacteria), water for breeding aquarium fish and so on.

In the paper and pulp industries, the fine-clustered water from pure water of the present invention can be used in digesting assistants, de-inking agents, pitch controller, anti-foaming agents, felt cleaner, paper strengthening agents, pigment dispersing agents, sizing agents, releasing agents, softening agents, slime controllers, anti-static agents, curling inhibitors and paper viscosity agents and for the improvement of water filtration.

In the cleaning industry, the fine-clustered water from pure water of the present invention can be used in cleaner compositions, cleaners for laundry, softening agents for laundry, solvents for dry cleaning, solvents for wet cleaning, and solvents for special cleaning.

In the metal/machinery/noble metal/jewel industries, the fine-clustered water from pure water of the present invention can be used in surface cleaners such as acid or alkali cleaners, solvents such as emulsifying solvents or emulsions, cleaners, anti-corrosive compositions, cleaners for precision instruments, cleaners for semiconductors and so on.

In the mining industry, the fine-clustered water from pure water of the present invention can be used in floatation concentrators such as foaming agents, collecting agents or absorbers of the collecting agents, coarse particulate eliminators, surface treatment preparations for ore, ignition inhibitors, heavy fluid stabilisers in dressings of ore or coal and so on.

In the leather industry, the fine-clustered water from pure water of the present invention can be used in the preliminary process (salting, soaking, liming, de-ashing, baking and de-fatting), tanning process (processing using plant tannin, chromium, mixed tannin and so on), coloring and fat addition processes, and finishing process (as a separating agent for use in mixing pigment with a wax glazing agent).

In the rubber/plastic industry, the fine-clustered water from pure water of the present invention can be used in synthetic rubber latex, adhesion inhibitors, mold release agents, lubricants, inorganic reinforcing agents, agents for polymerization (such as emulsion polymerization agents, suspension polymerization agents and bulk polymerization agents), lubricants used in processing (for example, external and internal activators for improving fluidity), dispersing agents of the filler, anti-static agents, surface modifiers of the plastic moldings, anti-clouding agents, slipping agents, release agents, anti-blocking agents and so on.

In the coloring material industry (such as pigments, paint, and ink), the fine-clustered water from pure water of the present invention can be used in surface pigment improvers, hydrophilic hydrophilization agents, lipophilization agents, dispersing agents for hydrophilic pigments in paint, dispersing agents for lipophilic pigments in paint, color development improvers, browning inhibitors, and aids thereof, additives thereof and so on.

In the civil engineering/construction industry, the fine-clustered water from pure water of the present invention can be used in admixtures of cement (for example, air entraining agents (AE agents) and aids thereof, wetting agents, dispersing agents, improvers for fluidity, foaming agents and so on), cutback asphalt (for example, peeling inhibitors and so on), asphalt emulsions, emulsifiers, coarse particulate depressants, sedimentary and coagulating agents, soil improvers, agents for rapid drying and so on.

In the ceramic industry, the fine-clustered water from pure water of the present invention can be used in additives such as aids for powdering agents, dispersing agents, binders and plasticizers, additives for glaze or muffle painting, oil lubricants, protective colloid agents, anti-foaming agents and so on.

In the coal and petroleum/fuel industries, the fine-clustered water from pure water of the present invention can be used in additives for COM, additives for CWS, coal dust inhibitors, detergents for mining petroleum, emulsion-destroying agents for crude oil, additives for fuel oil and so on.

In the photographic industry, the fine-clustered water from pure water of the present invention can be used in photosensitive emulsions (emulsions for couplers), anti-static agents which are added to the developing agent, dispersing agent of water-insoluble additives such as couplers and antiseptics in photographic emulsions, inhibitors for patches by drying, solvents for developer and fixer, and so on.

In the fermentation industry, the fine-clustered water from pure water of the present invention can be used in sterilizing agents, disinfectant, defoaming agents, productivity (yield) increasing agents, maturation promoting agents, fermentation promoting agents and so on.

Hereinafter, applications of the fine-clustered water from pure water of the present invention to pharmaceutical compositions, cosmetics (cosmetic creams, cosmetic lotions, hair liquid, foundation, dentifrice agents), shampoo and/or hair treatment, treatments, eye drop compositions, eye wash compositions, contact lens cleaner, and photo developing compositions are described. However, applications of the water of the present invention should not be limited to these areas.

Firstly, pharmaceutical compositions of the present invention are described. Since the pharmaceutical compositions of the present invention use fine-clustered water from pure water which has superior dispersing ability, the drugs are not only dissolved easier in the water but also absorbed easily by tissues, and therefore the drugs are rapidly carried or delivered to the desired site. Thus, there is no limitation to the drugs that can be used in the pharmaceutical compositions.

Pharmaceutical compositions of the present invention can be prepared in a dosage form suitable for the following administration and application: oral administration (gastrointenstinal tract), mucosal application (eye, oral mucosa, throat, nasal cavity, bronchus or lung, intestine, cavity of lower half of body), application to body surface (skin, body cavity, trauma), and intravascular or intratissue administration (intravenous, intramuscle and subcutaneous administration).

For oral administration, the dosage may take the form of a powder, granule, tablet for internal use, capsule, pill, elixir, syrup, limonade, aromatic water, emulsion, suspension, infusion, decoction, tincture for internal use, extract, or liquid extract.

For mucosa application, the dosage may take the form of an eye drop, eye ointment, and so on for the eye; sublingual tablet, buccal preparation, troche and so on for oral mucosa; gargles for throat; spray for nasal cavity; aerosol, inhalant and so on for bronchus or lung; enema and so on for intestine; and suppository, vaginal suppository and so on for the lower body cavity.

For body surface application, the dosage may take the form of an ointment, plaster, liniment, lotion, paste, cataplasm, liquid preparation, spirits, external tincture and so on for the skin. For the body cavity or trauma, drench and so on can be used.

For intravascular or intratissue administration, the dosage may take the form of an injection for subcutaneous use, intramuscular use, intravenous use and so on; and implants for subcutaneous tissue.

For preparation of the above dosage forms, vehicle, adjuvant, additives and so on which are generally used in the preparation of pharmaceuticals can be added. The water of the present invention can be used as the water component for these vehicle, adjuvant, additives and so on.

As the vehicle, solvents, bases, diluents, bulking agents, fillers, excipients and so on can be used.

As the adjuvant, solution adjuvants, solubilizers, buffering agents, tonicity agents, emulsifiers, suspending agents, dispersants, thickeners, gelling agents, stiffening agents, absorbents, adhesives, elastomers, plasticizers, binders, disintegrants, tablet lubricants, coating agents, sustained release agents, aerosol propellants and so on can be used.

As the additives, antioxidants, preservatives, humectants, light protecting agents, polishing agents, anti-static agents, aroma, sweetening agents, flavors, colorants, emollients, soothing agents and so on can be used.

As drugs suitable for use in pharmaceutical composition are, for example: drugs for the circulatory system such as cardiotonic, pressor, anti-arrhythmic agents, therapeutic drugs for angina, vasodilator, hypotensive drugs, diuretic agents, hyperlipemia and so on; drugs for the respiratory system sucn as bronchiectasis, respiratory facilitate, therapeutic drugs for bronchial asthma, ntitussive, expectorants and so on; drugs for the digestive system such as drugs for stomachic digestion, drugs for peptic ulcers, drugs for controlling intestinal function, cathartic drugs, therapeutic drugs for large intestine disease, therapeutic drugs for hemorrhoidal disease and so on; therapeutic drugs for liver/pancreas/gall bladder diseases such as therapeutic drugs for the liver, therapeutic drugs for the pancreas, antidotes, cholagogue and soon; drugs for acting on the urinary organs/genitalia such as therapeutic drugs for the kidney and so on; drugs for acting on the nervous system such as somnifacients, sedatives, antipyretic analgesics, antiepileptics, psychotropic agents, cerebral metabolic activators, drugs for the autonomic nerve system, therapeutic drugs for Parkinson's disease, muscle relaxants and so on; drugs for the vascular system such as hematopoietic agents, hemostats, anti-thrombotics and so on; therapeutic drugs for inflammation and allergies such as adrenocorticosteroids, non-steroid anti-inflammatory agents, anti-phlogistic enzymes, anti-rheumatic agents, antihistamine agents and so on; drugs acting on the metabolic system such as therapeutic drugs for diabetes mellitus, anti-podagrics and so on; drugs for pathogenic microorganisms; drugs for acting on sensitive organs such as ophthalmic remedies; drugs for otorhinology and dermatology; immunosuppressors; drugs for improving bone metabolism; hormone preparations; vitamin preparations; Chinese herbal remedies and so on.

The dosage form of pharmaceutical compositions can be prepared by any suitable method depending on their purpose.

Next, cosmetic creams of the present invention will be described. The cosmetic creams of the present invention include water which is obtainable by a fine clustering treatment of pure water and can disperse at least 1.5 times, preferably, at least two times as much glyceryl trioleate as purified water can. The fine-clustered water from pure water can disperse at most about five times as much glyceryl trioleate as purified water can.

The cosmetic creams generally include emollient cream, massage cream, cleansing cream, makeup cream, sunscreen cream, hair cream, deodorant cream, shaving cream, face cream, hand cream and body cream, which are classified by use, function, and site of application. In the use of the present invention, the cosmetic creams include all of the above creams.

The cosmetic creams can include, in addition to the water of the present invention, various components that are usually included in cosmetic creams such as an oil component, aqueous component, emulsifier and so on.

As the oil component, a natural oil component such as animal, plant or mineral oil or a synthetic oil component can be used.

As the oil component from an animal or plant, for example, bees wax, carnauba wax, candelilla wax, cacao butter, cetyl alcohol (1-hexadecanol), alkylstearate, lanolin, olive oil, camellia oil, caster oil, cotton oil, 9-octadecene-1-ol (oleil alcohol), oleic acid, squalane and so on can be used.

As the mineral oil component, solid paraffin, ceresin, microcryatalline wax, wax, vaseline, liquid paraffin, silicone oil and so on can be used.

As the synthetic oil component, synthetic ester oil, synthetic polyether oil and so on can be used.

The above oil component can be included in the cream, for example, from about 10 % to 80 % by weight. The content of the oil component depends on the types of creams such as oily, slightly oily, neutral and so on, and the amount to be added is determined accordingly.

As the aqueous component, humectants, viscosity-increasing agents, alcohol and so on can be used.

The humectants may contribute to the moisture of the keratin, feeling of use and so on. As the humectants, for example, glycerol, propylene glycol, dipropylene glycol, 1,3-butylene glycol, polyethylene glycol, polyhydric alcohol, sugars, amino acids and so on can be used. These humectants which can be added to the cosmetics are usually less than about 20 % by weight and preferably, less than about 15 % by weight.

The viscosity-increasing agent may contribute to moisture, stability and feeling of use. As the viscosity-increasing agent, for example, quince seed (quince seed gum), gum tragacanth, pectin, alginate, cellulose derivatives such as sodium carboxymethyl-cellulose, acrylic polymers, veegum, polyvinyl alcohol and so on can be used.

The alcohol may contribute by creating a refreshing feeling, sterilizing, dissolving the components and soon. As the alcohol, for example, ethanol, isopropanol and so on can be used.

Since the fine-clustered water from pure water used for cosmetic composition of the present invention has superior dispersing ability, the amount of addition of the above mentioned humectants, viscosity-increasing agents, alcohol and so on can be decreased.

The cosmetics using the present invention can contain appropriate amounts of other components such as softening agents (emollients), keratolytics, solubilizers, buffering agents, various drugs, perfume, antiseptics, dye, browning inhibitors, ultraviolet absorbers, metal ion blocking agents and so on.

The softening agents (emollients) may contribute to moisture, feeling of use and so on. As the softening agent, for example, ester oil, higher alcohols and so on can be used.

As the keratolytic, alkalis, for example, potassium hydroxide, potassium carbonate and so on, can be used.

As the emulsifier (solubilizing agent or surfactant), polyoxyethylene oleyl ether, octyl-dodecanol, lipophilic glycerol monostearate, self-emulsifying propylene glycol monostearate, polyoxyethylene cetyl ether, polyoxyethylenesorbitan monolaurylic acid ester and so on can be used. The emulsifier, for example, octyldodecanol, can be added in an amount of from about 2 % to 8 % by weight, preferably, from about 3 % to 5 % by weight. Since the water used for cosmetic composition of the present invention has superior dispersing ability, the amount added of the above mentioned emulsifiers can be decreased.

As for the drugs, astringents such as zinc p-phenolsulfonate and so on, sterilizing agents such as benzalkonium chloride and so on, nourishing agents such as vitamins, amino acid derivatives and so on can be used.

As a buffer (pH adjusting agent), citric acid and salt thereof can be used.

As stated above, the cosmetic cream of the present invention is superior in keeping moisture. The ability for keeping moisture can be measured as follows: a certain amount of the sample is uniformly spread to facies anterior antebrachii of subjects, for example, spreading density being 1.25 µl/cm²; after a certain period, the water content of the keratin layer is measured by high-frequency measuremeter of water in keratin.

Shampoo and treatments using the present invention may include, in addition to the fine-clustered water from pure water of the present invention, from about 1 % to 40 % by weight of surfactants, foam increasing agents, hydrotropes (solubilizer), emulsifying agents, conditioning agents, alcohol, dandruff removing agents, sterilizing agents, tonic agents, softening agents (emollients), keratolytics, viscosity-increasing agents, chelating agents, various drugs, perfume, antiseptic, dyes, browning inhibitors, ultraviolet absorbers, metal ion blocking agents and so on.

As a surfactant, any surfactant such as anionic, cationic, amphoteric and nonionic surfactant can be used. The surfactant can be contained in about 1 % to 40 % by weight. Since the fine-clustered water from pure water used in the shampoo or treatment is superior in dispersing activity, the amount of surfactant to be added can be decreased.

As the anionic surfactant, for example, alkylsulfate(AS), alkylether sulfate (AES), α-olefinsulfonate (AOS), soap, glycerin monoalkylester monosulfate, protein-fatty acid condensate, alkylphosphates and so on can be used.

The cationic surfactant can also be used as a main component of a hair rinse composition. As the cationic surfactant, for example, alkyltrimethylammonium chloride, dialkyldimethylammonium chloride, alkyldimethylbenzylammonium chloride and so on can be used.

As the amphoteric surfactant, for example, a surfactant such as imidazolin type, alkylbetaine type, alkylbetainesulfone type and so on can be used.

As the nonionic surfactant, for example, alkanolamide, amine oxide and so on can be used.

The foam increasing agent increases the foaming of the surfactant as a substrate of shampoo. As the foam increasing agent, alkanolamide of fatty acid, fatty acids, higher alcohol, water-soluble polymermolecules and so on can be used.

The viscosity-increasing agent may contribute to moisture, stability and feeling of use. As the viscosity-increasing agent, for example, quince seed (quince seed gum), gumtragacanth, pectin, arginate, cellulosederivatives (such as sodium carboxymethylcellulose), acrylic polymers, veegum, polyvinyl alcohol and electrolytes (such as sodium chloride, potassium chloride) and so on can be used.

The hydrotrope may contribute to low temperature stability of shampoo and to increased solubility of the surfactant. As the hydrotrope, for example, alcohol such as ethanol (which can also be used as a sterilizing agent), propylene glycol, glycerol (which can also be used as humectants), nonionic solbilizing agents such as polyoxyethylenesorbitan monolaurate and polyethyleneglycol ester of fatty acid, sodium benzenesulfonate, sodium xylenesulfonate, urea and so on can be used.

The emulsifying agent emulsifies the shampoo and may contribute to a feeling of high class, mildness and creaminess of the shampoo. As the emulsifying agent, for example, styrene polymer, polyvinylacetate and so on can be used. Manganese stearate, etyleneglycol monostearate, ethyleneglycol distearate, stearyl alcohol, fish scale flakes, titaneted mica and so on can be used as pearl brightners.

The conditioning agent may contribute to ease of combing, improvement of dry, loose hair and wiry hair, ease of arrangement, strengthening of hair and so on. As the conditioning agent, for example, substances having chemical absorbability such as hydrolysate of collagen, amino acids, sodium 2-pyrrolidone-5-carbonic acid, milk casein, ovalbumin, lecithin, pantenol (vitamin B complex) and so on; lanolin, squalane, and derivatives thereof; liquid paraffin, vaseline, fatty acid, higher alcohol, ester, silicone oil and derivatives thereof; oily substance having physical absorbability such as oil and fats (for example, castor oil and olive oil); ionic absorbable cationic polymers such as cationic denatured cellulose ether derivatives, quaternary ammonium salt of polyvinylpyrrolidone derivatives, chlorinated distearyldimethylammoniumpolymer, alkylpolyethylene imine and so on can be used.

The alcohol may contribute by creating a refreshing feeling, sterilizing, dissolving the components and soon. As the alcohol, for example, ethanol, isopropanol and so on can be used.

As the dandruff removing agent, for example, sulfur compounds such as sulfur, selenium sulfide, Zpt and so on, menthols, triclosan, halocarban, allantoin, salicylic acid and so on can be used.

The humectants may contribute to moisture, feeling of use and so on. As the humectants, for example, glycerol, propyleneglycol, dipropyleneglycol, 1,3-butyleneglycol, polyethyleneglycol, polyalcohol, sugars, amino acids, sorbitol and so on can be used.

The softening agent (emollients) may contribute to moisture, feeling of use and so on. As the softening agent, for example, ester oil, higher alcohol and so on can be used.

As the keratolytic, alkalis such as potassium hydroxide and potassium carbonate can be used.

As the drugs, astringents such as zinc p-phenolsulfonate and so on, sterilizing agents such as benzalkonium chloride and so on, nourishers such as vitamins and/or amino acid derivatives and so on can be used.

As a buffer agent (pH adjusting agent), citric acid and salts thereof can be used.

Since the fine-clustered water from pure water of the present invention used in the cosmetic compositions has superior dispersing ability, the additive amounts of the aforementioned viscosity-increasing agents, alcohol and so on can be decreased. For producing cosmetics, shampoo, hair treatment and/or hair liquid with the present invention, any suitable methods can be used.

Next, the eye drop composition will be described. The eye drop composition of the present invention can include, in addition to the fine-clustered water from pure water, many kinds of components which are usually blended in eye drop compositions such as angiotonics, vitamins, cell activating agents, parasympathmimetics, antiphlogistics, antihistamines, sulfa drugs, cornea protecting agents, tear components, disinfectant, transparent keeping agents, and so on. Usually, an eye drop composition contains about 99 % by weight of water and about 1 % by weight of tear components and other components.

As the angiotonic, for example, ephedrine hydrochloride and naphazoline hydrochloride can be used.

As the vitamins, for example, vitamin B2 complex, vitamin B6 composition, vitamin B12 composition, pantothenic acid and derivatives thereof and vitamin E can be used.

As the cell activating agent, for example, L-aspartic acid, allantoin, taurine and so on can be used.

As the parasympathmimetic, for example, neostigmine methylsulfate and so on can be used.

The antiphlogistic depresses allergic reaction. As the antiphlogistic, for example, dipotassium glycyrrhizinate, tetrahydrozoline hydrochloride, lysozyme chloride, sodium azulenesulfonate and so on can be used.

As the antihistamines, for example, chlorpheniramine maleate, dipotassium glycyrrhizinate, and diphenhydramine hydrochloride can be used.

The sulfa drug has anti-inflammatory action and anti-biotic action and therefore is effective with sties, bleary eyes and eye discharges. As the sulfa drug, sulfamethoxazole, sodium sulfamethoxazole, sodium sulfisomidine and so on can be used.

As the cornea protecting agent, for example, sodium chondroitin sulfate can be used.

The tear component has functions which depress inflammation and protect the eyeball and cornea. As the tear component, for example, sodium chloride, potassium chloride, calcium chloride can be used. Sodium chloride can be included from about 0.5 % to about 0.75 % by weight. Potassium chloride can be included from about 0.07 % to about 0.2 % by weight. The sum of these two components may be from about 0.7 % to about 1.0 % by weight.

As a disinfectant, polyvinyl alcohol can be used.

The eye drop composition of the present invention has superior drug penetration and absorption ability, so that highly effective treatment of an eye disease can be obtained.

Next, contact lens cleaners of the present invention will be described. The contact lens cleaner contains water, enzymes such as proteolytic enzymes, surfactants, disinfectants, and other additives. Preferably, the contact cleaner contains from about 80 % to about 99.9 % by weight of the water of the present invention and contains proteolytic enzymes and other necessary ingredients. As the other necessary ingredients, NaCl can be contained from about 0.5 % to about 0.75 % by weight and KCl can be contained from about 0.07 % to about 0.2 % by weight. The sum of these two components can be contained from about 0.7 % to about 1.0 % by weight. The contact lens cleaner of the present invention is superior in affinity between the ingredients, and can be stored for a long time without forming any precipitate.

As the enzymes, various kinds of protease, proteolytic enzymes such as papain, mucin-decomposing enzyme, oil-and fat-decomposing enzymes such as lipase, and hyaluronic acid-decomposing enzymes such as hyaluronidase can be used.

As a surfactant, a nonionic surfactant is preferably used. As the disinfectant, polyethylene glycol and so on can be used.

As the other additives, EDTA-2Na, NaCl, boric acid, borax, sodium carbonate anhydride, sodium bicarbonate, and citric anhydride can be used.

Since the contact lens cleaner of the present invention includes water with a superior dispersing ability, the amount of additives such as surfactants, disinfectants, and so on can be decreased. Further, the contact lens cleaner using the present invention can attain the effect that enzyme action in the cleaner is improved.

### Examples

Hereinafter, the present invention will be explained using examples; however, the present invention is not limited to these examples.

### (Example 1: production of the water of the present invention)

The fine-clustered water was obtained by using the above-mentioned apparatus. The width of the water channel (slit) was 2mm and the rate of the water stream was about 15m/min. The circulating water was irradiated with magnetic field of intensity of 1.2T for 24 hours. The water used at the start was pure water or purified water. The thus-obtained the fine-clustered water from pure water, the fine-clustered water from purified water and purified water were subjected to ¹⁷O-NMR measurement by the use of JNM EX-270 (Japan Electron Optics Laboratory Ltd.(JEOL. Ltd.)). The half width of ¹⁷O-NMR signal at 20°C were as follows:

| | |
|---|---|
| the fine-clustered water from pure water | 56.3Hz |
| the fine-clustered water from purified water | 83.7Hz |
| purified water | 142.4Hz |

### (Example 2: ability to disperse glyceryl trioleate)

To each 10ml of the fine-clustered water from pure water, the fine-clustered water from purified water and purified water, 43.5mg of glyceryl trioleate were added, followed by shaking at 128rpm for 5 minutes. Immediately after the shaking, 4ml samples were collected. Each 0.54ml was put in a NMR sample tube with diameter of 5mm. To this tube, 0.06ml of 10mM TSP-d₄ in deuterium solution was added as an internal standard so that the final concentration of TSP-d₄ became 1mM and ¹H-NMR was measured. In this measurement, since the large ¹H signals by water are not necessary, the ¹H-NMR spectrum was measured by 128 times accumulated measurement by a ¹H Homo Gated Decoupling (HMG) method. The concentration of the dispersed glyceryl trioleate in the water was calculated from the ratio of the signal strength of the methylene group of the glyceryl trioleate and the signal intensity of the methyl proton of TSP-d₄, an internal standard. The measurement was performed using JNM LA-400 FT-NMR Spectrometer of JEOL. Ltd.

From the measurement, the relative intensity of the glyceryl trioleate dispersed in the fine-clustered water from pure water was about two times as much as that of purified water. Also, it was found that the relative intensity of the glyceryl trioleate dispersed in the fine-clustered water from purified water was only 1.1 times as much as that of purified water. These facts indicated that the fine-clustered water has a superior ability to disperse glyceryl trioleate to that of the purified water.

### (Example 3: ability to disperse glyceryl monooleate)

Glyceryl monooleate was dispersed in the fine-clustered water from pure water, the fine-clustered water from purified water and purified water in the same manner as example 2, and observed by human eyes. In the case of purified water, phase separation was observed within 5 minutes; however, in the cases of the fine-clustered water from pure water and the fine-clustered water from purified water, an emulsion was retained for 24 hours and 2 hours, respectively, without phase separation. This shows that the fine-clustered water from pure water of the present invention has a superior oil-dispersing ability.

### (Example 4: absorbing test of ethanol)

Fifteen mature whister rats of seven weeks of age were prepared. Ethanol was dissolved in the fine-clustered water from pure water so that the concentration of ethanol was 16 % (the Example alcohol solution). As a comparative example, a 16 % ethanol solution was prepared in the fine clustered water from purified water (the Comparative Example alcohol solution). As a control, a 16% ethanol solution in purified water (the Control alcohol solution) was used. To each 5 rats, the Example alcohol solution, the Comparative Example alcohol solution and the Control alcohol solution were injected into the abdominal cavity at the ratio of 0.5g/Kg, respectively. Blood samples were collected at 30, 60, 90, 150 and 210 minutes after injection, respectively, and serum of each sample was prepared. The alcohol concentration of each sample was measured by ¹H-NMR.

NMR was measured using JNM-EX400 (400MHz,9.3T) FT-NMR. Ethanol concentration (mM) was calculated by using 1mM dimethylsulfoxoide(DMSO) as an internal standard. The wide ¹H-NMR signals from protein were repressed by using the Carr-Purcell-Meiboom Gill pulse series for T₂ measurement, and the signal of water was repressed by the combination of the C-P-M Gill series and the Homo Gated Decoupling method, and was accumulated 16 times at intervals of 100 milisecond, repeat 15 seconds, at 30 °C.

The result is shown in Figure 1. In Figure 1, symbols ○, ● and □ show the Example alcohol solution, the Comparative Example alcohol solution and the Control alcohol solution, respectively. This result shows that ethanol dissolved in the fine-clustered water from pure water was absorbed about 1.3 times faster than that in purified water, and about 1.2 times faster than that in the fine-clustered water from purified water.

### (Example 5)

A cosmetic cream (cream type) having below-indicated composition was prepared as follows:

To the fine-clustered water from pure water obtained in Example 1, glycerol, propylene glycol and potassium hydroxide were added, and then mixed and stirred under heat (70°C) so as to obtain an aqueous component.

As well, stearic acid, butyl stearate and stearyl alcohol were mixed and melted by heating to obtain an oil component which was kept at 70°C. Then, perfume, antiseptic and an antioxidant were added to this melted oil component, stirred and mixed sufficiently so as to obtain an oily solution. Then, this oily solution and the above aqueous component were mixed and stirred at 70°C to obtain a cosmetic cream.

### (Composition of the cosmetic cream)

In the composition, the percent is based on percent by weight.

| Aqueous component: | |
|---|---|
| water | 60.5% |
| alkali: potassium hydroxide | 0.5% |

| oil component: | |
|---|---|
| stearic acid | 10% |
| stearyl alcohol | 4% |
| butyl stearate | 8% |

| humectant: | |
|---|---|
| propylene glycol | 10% |
| glycerol | 4% |

| emulsifier: | |
|---|---|
| glycerol monostearate | 3% |
| perfume, antiseptic, antioxidant | small amounts |
| | (total 100%) |

The components of this cosmetic cream were still well emulsified and dispersed in the cosmetic cream even after one year from preparation. When the cosmetic cream was spread on the skin, the cream did not stick and was smooth and soft. The feeling of using this cream was good. Its ability to retain moisture was also superior.

### (Comparative example 1)

A cosmetic cream (cream type) was prepared in the same manner as that of example 5 except that purified water was used instead of the fine-clustered water from pure water.

When the thus-obtained cosmetic cream was observed 30 days from preparation, the dispersing ability of the components was not good and the liquid was apt to separate from the emulsion.

### (Example 6)

A cosmetic cream (cream type) having below-indicated composition was prepared as follows:

To the fine-clustered water from pure water obtained in Example 1, glycerol, propylene glycol and triethanolamine were added so as to obtain an aqueous component.

As well, bees wax, squalane, stearic acid and stearyl alchol were mixed to obtain an oil component which was kept at 70°C. Then, perfume, antiseptic and an antioxidant were added to this oil component, stirred and mixed sufficiently so as to obtain an oily solution. Then, this oily solution and the above aqueous component were mixed and stirred at 70°C so as to obtain a cosmetic cream.

### (Composition of the cosmetic cream)

In the composition, the percent is based on percent by weight.

| Aqueous component: | |
|---|---|
| water | 57.5% |
| alkali: triethanolamine | 1% |

| oil component: | |
|---|---|
| bees wax | 2% |
| stearic acid | 8% |
| stearyl alchol | 5% |
| squalane | 10% |

| humectant: | |
|---|---|
| propylene glycol | 8% |
| glycerol | 4% |

| emulsifier: | |
|---|---|
| self emulsifying propylene | |
| glycol monostearate | 3% |
| polyoxyethylenecetylether | 1% |
| perfume, antiseptic, antioxidant | small amounts |
| | (total 100%) |

The components of this cosmetic cream were still well emulsified and dispersed in the cosmetic cream even after one year from preparation. When the cosmetic cream was spread on the skin, the cream did not stick and was smooth and soft. The feeling of using this cream was good. Its ability to retain moisture was also superior.

### (Example 7)

A cosmetic cream (cream type) having below-indicated composition was prepared as follows:

To the fine-clustered water from pure water obtained in Example 1, borax was added, stirred, and mixed and retained at 70°C under heat so as to obtain an aqueous component.

As well, bees wax and liquid paraffin were mixed and melted with heat and kept at 70°C so as to obtain an oil component. Then, this melted oil component and the above aqueous component were mixed and stirred at 70°C so as to obtain a cosmetic cream.

### (Composition of the cosmetic cream)

In the composition, the percent is based on percent by weight.

| Aqueous component: | |
|---|---|
| water | 39% |
| borax | 1% |

| Oil component: | |
|---|---|
| bees wax | 10% |
| liquid paraffin | 50% |
| | (total 100%) |

The components of this cosmetic cream were still well emulsified and dispersed in the cosmetic cream even after one year from preparation. When the cosmetic cream was spread on the skin, the cream did not stick and was smooth and soft. The feeling of using this cream was good. Its ability to retain moisture was also superior.

### (Example 8)

A cosmetic cream (milk lotion type) having below-indicated composition was prepared as follows:

To the fine-clustered water from pure water obtained in Example 1, glycerol, propylene glycol, and triethanolamine were added, stirred, and mixed, and the thus-obtained aqueous component was retained at 70°C under heat.

As well, stearic acid, cethanol, vaseline, lanolin alcohol and liquid paraffin were mixed and melted with heat and kept at 70°C so as to obtain an oil component. Then, perfume, antiseptic and an antioxidant were added to this oil component, stirred and mixed sufficiently so as to obtain an oily solution.

Then, this oily solution and the above aqueous component were mixed and stirred at 70°C so as to obtain a cosmetic cream (milk lotion type).

### (Composition of the cosmetic cream (milk lotion type))

In the composition, the percent is based on percent by weight.

| Aqueous component: | |
|---|---|
| water | 70% |
| glycerol | 3% |
| propylene glycol | 5% |
| triethanolamine | 1% |

| Oil component: | |
|---|---|
| stearic acid | 2% |
| cethanol | 1.5% |
| vaseline | 3% |
| lanolinalcohol | 2% |
| liquid paraffin | 10% |
| perfume, antiseptic, antioxidant | small amounts |
| | (total 100%) |

The components of this cosmetic cream were still well emulsified and dispersed in the cosmetic cream even after one year from preparation. When the cosmetic cream was spread on the skin, the cream did not stick and was smooth and soft. The feeling of using this cream was good.

### (Example 9)

A cosmetic lotion having below-indicated composition was prepared as follows:

To the fine-clustered water from pure water obtained in Example 1, glycerol, propylene glycol and ultraviolet absorber were added, stirred, and mixed at an ambient temperature (25°C).

As well, an appropriate amount of softening agent (emollient), surfactant, antiseptic and perfume were added to ethanol and mixed. Then, the mixture was mixed with the above aqueous mixture so as to prepare a cosmetic lotion.

### (Composition of the cosmetic lotion)

In the composition, the percent is based on percent by weight.

| Aqueous component: | |
|---|---|
| water | 79.7% |

| humectant : | |
|---|---|
| glycerol | 6% |
| propylene glycol | 4% |

| softening agent: | |
|---|---|
| oleil alcohol | 0.1% |

| surfactant: | |
|---|---|
| polyoxyethylene lauryl ether | 1% |
| ethanol | 10% |
| perfume, antiseptic, antioxidant | small amounts |
| | (total 100%) |

When the cosmetic lotion was spread on the skin, the lotion did not stick and was soft. The feeling of using this lotion was good.

### (Example 10)

A foundation (cream type) having below-indicated composition was prepared by using the fine-clustered water from pure water obtained in Example 1.

### (Composition of the foundation)

In the composition, the percent is based on percent by weight.

| | |
|---|---|
| water | 54.5% |
| triethanolamine | 1.2% |
| sorbitol | 2% |
| methyl p-oxybenzoate | small amount |
| pigment:(titanium oxide, talc, kaolin) and perfume | 15.5% |
| stearic acid | 5% |
| lipophilic glycerol monostearate | 2.5% |
| cetostearylalcohol | 1% |
| propyleneglycol monolaurate | 3% |
| liquid paraffin | 7% |
| isopropylmyristate | 8% |
| butyl p-oxybenzoate | small amount |
| | (total 100%) |

When this foundation was spread on the skin, the foundation did not stick and was soft. The feeling of using this foundation was good.

### (Example 11)

A hair liquid having below-indicated composition was prepared by using the fine-clustered water from pure water obtained in Example 1.

### (Composition of the hair liquid)

In the composition, the percent is based on percent by weight.

| | |
|---|---|
| water | 31% |
| polyoxypropylenebutylether | 13% |
| ethanol | 56% |
| disodium edetate | small amount |
| perfume, dye | small amounts |
| | (total 100%) |

When this hair liquid was used on the hair, the hair did not stick. It felt refreshing.

### (Example 12)

A shampoo (transparent type) having below-indicated composition was prepared by using the fine-clustered water from pure water obtained in Example 1.

### (Composition of the shampoo)

In the composition, the percent is based on percent by weight.

| | |
|---|---|
| water | 76% |
| sodium alkylethersulfate | 16% |
| diethanolamide laurirate | 4% |
| propylene glycol | 2% |
| dye, antiseptic and ultraviolet absorber | each appropriate amount |
| | (total 100%) |

The components of this shampoo were still well emulsified and dispersed in this shampoo even after one year from preparation. When the shampoo was used, the hair was soft. The feeling of using this shampoo was good.

### (Comparative example 2)

Shampoo was prepared in the same manner as that of example 12 except that purified water was used instead of the fine-clustered water from pure water.

When the thus-obtained shampoo was observed 30 days from preparation, the dispersion condition of the components was bad. When the shampoo was used, the hair was dry and loose. The feeling of use was bad.

### (Example 13)

A shampoo (pearl type) having below-indicated composition was prepared by using the fine-clustered water from pure water obtained in Example 1.

### (Composition of the shampoo)

In the composition, the percent is based on percent by weight.

| | |
|---|---|
| water | 76% |
| alkylsulfate triethanolamine | 15% |
| coconut fatty acid monoethanolamide | 5% |
| ethyleneglycolmonostearate | 2% |
| perfume, dye, antiseptic | appropriate amounts |
| | (total 100%) |

The components of this shampoo were still well emulsified and dispersed in this shampoo even after one year from preparation. When the shampoo was used, the hair was soft. The feeling of using this shampoo was good.

### (Example 14)

A shampoo (conditioning type) having below-indicated composition was prepared by using the fine-clustered water from pure water obtained in Example 1.

### (Composition of the shampoo)

In the composition, the percent is based on percent by weight.

| | |
|---|---|
| water | 77% |
| alkylether aminesulfate | 18% |
| coconut fatty acid diethanolamide | 2% |
| cationic denatured cellulose ether | 1.5% |
| perfume, dye, antiseptic | appropriate amounts |
| | (total 100%) |

The components of this shampoo were still well emulsified and dispersed in this shampoo even after one year from preparation. When the shampoo was used, the hair was soft. The feeling of using this shampoo was good.

### (Example 15)

A rinse having below-indicated composition was prepared by using the water obtained in Example 1.

### (Composition of the rinse)

In the composition, the percent is based on percent by weight.

| | |
|---|---|
| water | 96% |
| chlorinated stearyldimethylbenzylammonium | 1.4% |
| stearyl alchol | 0.6% |
| glycerine monostearate | 1.5% |
| sodium chloride | 0.2% |
| perfume, dye, antiseptic | appropriate amounts |
| | (total 100%) |

The components of this rinse were still well emulsified and dispersed in this shampoo even after one year from preparation. When this rinse was used, the hair was soft. The feeling of using this shampoo was good.

### (Example 16)

A contact lens cleaner having below-indicated composition was prepared by using the fine-clustered water from pure water obtained in Example 1.

### (Composition of the contact lens cleaner)

In the composition, the parts are based on weight.

| | |
|---|---|
| water | 100 parts |
| papain and neutral protease | 18.5 parts |
| lipase | 1.4 parts |
| hyaluronidase | 1.4 parts |
| lecithinase | 1.4 parts |
| nonionic surfactant | 2.2 parts |
| EDTA-2Na | 4.3 parts |
| Sodium chloride | 12.9 parts |
| sodium bicarbonate and citric anhydride | 37.6 parts |
| sodium carbonate anhydride | 6.5 parts |
| borax | 8.6 parts |
| polyethylene glycol | 5.2 parts |

The components of the thus-obtained contact lens cleaner were homogeneously mixed even after one year from preparation, and it was able to be preserved for a long time without causing any precipitation. The amount of non-ioninc surfactant used was very low compared with that of conventional cleaner.

### (Comparative example 3)

A contact lens cleaner was prepared in the same manner as that of example 16 except that purified water was used instead of the fine-clustered water from pure water.

When the thus-obtained contact lens cleaner was observed after 30 days from preparation, the dispersion condition of the components was bad and precipitate was observed.

### (Example 17)

Eye drops having below-indicated composition were prepared by using the fine-clustered water from pure water obtained in Example 1.

### (Composition of the eye drops)

In the composition, the percent is based on percent by weight.

| | |
|---|---|
| water | 99.0% |

| tear component: | |
|---|---|
| sodium chloride | 0.7% |
| potassium chloride | 0.2% |

| antiphlogistic: | |
|---|---|
| sodium azulenesulfonate | 0.02% |
| tetrahydrozoline hydrochloride | 0.02% |

| antihistamine | |
|---|---|
| chlorpheniramine maleate | 0.03% |

The components of the thus-obtained eye drop composition were homogeneously mixed even after one year from preparation, and it was able to be preserved for a long time without causing any precipitation.

### (Example 18)

A dentifrice having below-indicated composition was prepared by using the fine-clustered water from pure water obtained in Example 1.

### (Composition of the dentifrice)

In the composition, the percent is based on percent by weight.

| | |
|---|---|
| water | 43% |
| calcium carbonate | 39% |
| sorbitol | 15% |
| sodium carboxymethylcellulose | 0.6% |
| sodium laurylate | 1.3% |
| saccharin | 0.1% |
| perfume(mint), presevative | small amounts |
| | (total 100%) |

The components of the thus-obtained dentifrice were homogeneously mixed, and the feeling of use was good. The amount of sodium laurylate used can be decreased.

### (Example 19)

A developing solution and fixing solution for monochrome photography were prepared by using the fine-clustered water from pure water obtained in Example 1 and purified water as a control. The developer and the fixing agent were purchased from Fuji Film Company Limited (Japan). The developing solution and fixing solution were prepared according to the manufacturer's indication. The photographs developed and fixed by using the fine-clustered water from pure water were very clear, which could be recognized even by human eyes as compared with those developed using purified water. When these photographs were observed by the stereoscopic microscope, there was a significant difference in the clearness between them.

### (Effects of the present invention)

The fine-clustered water from pure water of the present invention has a superior ability to disperse oil and fats and superior absorbancy by cells. Thus, the water of the present invention has the effect that, in a pharmaceutical field, drugs can be rapidly carried or delivered. The water of the present invention having the above features can also be applied to the food and cosmetic fields.

Various other modifications will be apparent to, and can be readily made by, those skilled in the art without departing from the scope and spirit of this invention. Accordingly, it is not intended that the scope of the claims appended hereto be limited to the descriptions as set forth herein, but rather that the claims be broadly construed.

Fine-clustered water made from pure water is obtained by fine clustering treatment of pure water and is able to disperse at least about 1.5 times as much glyceryl trioleate as purified water is.

The water of the present invention can be used in pharmaceutical compositions, food compositions, cosmetic compositions, eye drop compositions, eye wash compositions, contact lens cleaner, agricultural chemical compositions, photo developing solution, and concrete compositions.

Especially, when the fine-clustered water of the present invention is used in pharmaceutical compositions, not only are the drugs dissolved easily in the water, but they are also rapidly absorbed via and delivered to tissue (oral mucosa, skin, mesentery) as compared with the purified water.

## Claims

1. Fine-clustered water from pure water which is obtained by fine clustering treatment of pure water and is able to disperse at least about 1.5 times as much glyceryl trioleate as purified water is.

2. The fine-clustered water from pure water according to claim 1, wherein the fine-clustered water from pure water is able to disperse at least about 2 times as much glyceryl trioleate as purified water is.

3. The fine-clustered water from pure water according to claim 1, wherein the fine-clustered water from pure water is obtained by treating pure water with magnetic field.

4. A method for producing the fine-clustered water from pure water which is able to disperse at least about 1.5 times as much glyceryl trioleate as purified water is, wherein the method comprised the step of a fine clustering treatment of pure water.

5. The method according to claim 4, wherein the fine clustering treatment is treatment with magnetic field.

6. A composition which comprises the fine-clustered water from pure water which is obtainable by fine clustering treatment of pure water, wherein the fine-clustered water from pure water is able to disperse at least about 1.5 times as much glyceryl trioleate as purified water is.

7. The composition according to claim 6, wherein the fine-clustered water from pure water is able to disperse at least about 2 times as much glyceryl trioleate as purified water.

8. The composition according to claim 6, wherein the fine-clustered water from pure water is obtained by treating pure water with magnetic field.

9. The composition according to claim 6, wherein the composition is a pharmaceutical composition, cosmetic, food composition, eye drop composition, eye wash composition, contact lens cleaner, agricultural chemical composition, photo developing solution, or concrete composition.

10. The composition according to claim 9, which is a pharmaceutical composition and wherein the absorption speed of a drug in the pharmaceutical composition via transdermal, mucosa or mesentery is faster than that of the case where the drug is in a pharmaceutical composition based on purified water.

11. The composition according to claim 9, which is a food composition for beverage use.

12. The composition according to claim 9, which is a cosmetic composition selected from the group consisting of cosmetic creams, cosmetic lotions, foundation, shampoo, hair treatment and hair liquid.
